# EUROPÄISCHE PATENTANMELDUNG

(11) **EP 1 366 673 A2**
(43) Veröffentlichungstag der Anmeldung: **03.12.2003**
(21) Anmeldenummer: 03011067.0
(22) Anmeldetag: 20.05.2003
(51) Int. Cl.: A23K 1/16, A61K 35/78, A23L 1/0526

(54) **Entzündungshemmendes und chemopräventives Mittel auf der Basis von Johannisbrotprodukten**

(30) Priorität: 28.05.2002 DE 10223856
(71) Anmelder: Nutrinova Nutrition Specialties & Food Ingredients GmbH, 65929 Frankfurt am Main (DE)
(72) Erfinder: Haber, Bernd, Dr., 55126 Mainz (DE)
(74) Vertreter: Schweitzer, Klaus, Dr.

(57) **Zusammenfassung**

Die Erfindung betrifft ein entzündungshemmendes und chemopräventives Mittel, welches Produkte aus Johannisbrot, insbesondere Johannisbrotfaser, enthält. Weiterhin betrifft die Erfindung ein Verfahren zur Herstellung dieser Mittel sowie deren Verwendung mit dem Ziel der Absenkung des Risikos, der Verhinderung, der Vorbeugung und der Behandlung von Entzündungskrankheiten oder Krebserkrankungen, insbesondere des Gastrointestinaltraktes.

## Beschreibung

Die Erfindung betrifft ein entzündungshemmendes und chemopräventives Mittel, welches Produkte aus Johannisbrot, insbesondere Johannisbrotfaser, enthält. Weiterhin betrifft die Erfindung ein Verfahren zur Herstellung dieser Mittel sowie deren Verwendung mit dem Ziel der Absenkung des Risikos, der Verhinderung, der Vorbeugung und der Behandlung von Entzündungskrankheiten oder Krebserkrankungen, insbesondere des Gastrointestinaltraktes.

Eine Entzündung ist eine Abwehrreaktion des Organismus auf innere oder äußere Reize (z. B. Druck, Fremdkörper, Strahlen, Mikroorganismen oder krankhafte Stoffwechselprodukte). Hierbei kann es zu Rötung, Hitze, Schwellung, Schmerzen und Funktionsstörungen kommen. Die Entzündungsreaktion geht vom Blutgefäßbindegewebe des geschädigten Organs oder Gewebes aus. Durch Vermittlung hormonähnlicher Wirkstoffe werden verschiedene Abwehrsysteme des Körpers aktiviert.

Entzündliche Erkrankungen des Magen-Darmbereiches umfassen u. a. Darmentzündungen (Enteritis), Gastroenteritis, unspezifisches Reizdarmsyndrom und die chronisch entzündlichen Darmerkrankungen Colitis ulcerosa und Morbus Crohn.

Chronisch entzündliche Darmerkrankungen äußern sich über Symptome wie Stuhlunregelmäßigkeiten, Durchfall, Bauchschmerzen, Übelkeit oder Gewichtsabnahme. Bei den chronisch entzündlichen Darmerkrankungen unterscheidet man normalerweise Colitis ulcerosa und Morbus Crohn, die unterschiedliche Ernährungs- und Behandlungsmaßnahmen nach sich ziehen. Während Morbus Crohn sich über alle Abschnitte des Verdauungstrakts ausbreiten kann und diskontinuierliche Entzündungsherde hinterlässt, ist die Colitis ulcerosa auf den Dickdarmbereich beschränkt und dehnt sich kontinuierlich vom Rektum aus. Problematisch ist, dass Ernährungsmaßnahmen vom Individuum, aber auch vom Zeitpunkt innerhalb des jeweiligen Krankheitsverlaufs abhängen. Als Auslöser dieser Erkrankungsarten werden infektiöse Faktoren (wie Viren, Bakterien oder Pilze), genetische Faktoren, diätetische Faktoren (z. B. Zucker, Eiweiß), psychische Faktoren und eine Überreaktion der Immunabwehr diskutiert. Chronische Entzündungskrankheiten über viele Jahre erhöhen massiv das Risiko, an Krebs zu erkranken.

Krebs ist ein multifaktorielles Erkrankungsbild, das durch verschiedene Faktoren verursacht, aber auch beeinflusst werden kann. Es wird vermutet, dass bestimmte Krebserkrankungen durch richtige Ernährung und einen gesunden Lebensstil vermieden werden können. Insbesondere Krebsarten des Gastrointestinaltraktes wie Dickdarmund Magenkrebs gehören zu den am stärksten durch Ernährung beeinflussbaren Krebserkrankungen.

Zu Beginn des Kanzerogeneseprozesses steht in den meisten Fällen der Kontakt der DNA mit kanzerogenen Substanzen. Diese können endogen entstehen oder exogen zugeführt werden. Als endogene Faktoren können physiologisch gebildete Stoffe eine Rolle spielen, die die DNA schädigen können, als exogene Faktoren können neben Viren oder radioaktiver Strahlung v.a. sogenannte Prokanzerogene genannt werden, die erst nach metabolischer Aktivierung genotoxisch sind. Auch Einflussfaktoren auf die Aktivität von Entgiftungsenzymen oder DNA-Reparaturenzymen können entscheidenden Einfluss auf das Krebsgeschehen haben. Nahrungsbestandteile können in verschiedenster Art und Weise in die Krebsentstehung positiv eingreifen. So zeigen z. B. bestimmte Vitamine wie Vitamin C oder Vitamin E oder auch bestimmte Pflanzeninhaltsstoffe wie Carotenoide oder Flavonoide Eigenschaften, die mögliche Schädigungen durch krebsverursachende Substanzen verhindern können.

Kolorektale Karzinome gehören zu der am häufigsten auftretenden Krebsart des Magen-Darmtraktes. Als wichtigste Einflussfaktoren dieser Krebserkrankungen gelten die Ernährung und körperliche Aktivität. Entzündungsreaktionen gelten als risikofördernd, während Acetylsalicylsäure oder andere nicht-steroidale Entzündungshemmer einen protektiven Effekt haben. Der Anteil an erblich bedingten kolorektalen Karzinomen wird auf weit unter 10% geschätzt.

Bisher hat man entzündliche Erkrankungen des Magen-Darmtraktes v.a mit entzündungshemmenden Arzneimitteln wie Kortikosteroiden, aber auch Antibiotika und Antidiarrhoika behandelt. Generell sind die Kortikosteroide die wirksamsten Medikamente zur Behandlung von akuten Schüben von chronisch entzündlichen Darmerkrankungen, allerdings können sie nicht über längere Zeit eingesetzt werden. Die Langzeittherapie mit systemisch wirkenden Kortikosteroiden führt zu Nebenwirkungen, die häufig zur Verringerung der Dosis bzw. zum Abbruch der Therapie zwingen. Die Nebenwirkungen beinhalten u. a. Gewichtszunahme mit Stammfettsucht, Mondgesicht, Akne, Nebennierenrinden-Schrumpfung, Erhöhung von Blutzucker und Cholesterin, Blutdruckanstieg oder Osteoporose.

Bei leichten bis mäßigen Schüben der Colitis Ulcerosa oder bei Erhaltung der Beschwerdefreiheit, aber auch bei speziellen Formen wie der linksseitigen Colitis ulcerosa wird bevorzugt 5-Aminosalicylsäure benutzt. Auch hier ist bei Langzeittherapien mit möglichen Nebenwirkungen wie z. B. Blutbildveränderungen oder eine Beeinträchtigung der Nierenfunktion, aber auch Übelkeit, Erbrechen, Durchfall und Bauchschmerzen zu rechnen.

Neben der medikamentösen Therapie sind Ernährungsmaßnahmen wichtig. Diese versuchen in erster Linie die durch die Entzündungserkrankungen auftretende Unterversorgung mit Nährstoffen aufzufangen und dieser entgegenzuwirken. Als Nahrungsmittelinhaltsstoffe, die einen positiven Einfluss auf das Krankheitsbild haben können, sind viskose, gut quellende Ballaststoffe wie z. B. Pektin, Haferkleie, Guar oder Psylliumsamenmehl zu nennen. Weiterhin scheinen mehrfach ungesättigte Fettsäuren eine hemmende Wirkung bei bestimmten Entzündungskrankheiten zu haben. Auch wird ein positiver Einfluss von Probiotika bei entzündlichen Darmerkrankungen diskutiert. Diese Nahrungsmittelbestandteile wurden bisher v.a. unterstützend innerhalb bestimmter Diätmaßnahmen empfohlen, wobei meist empirisch ermittelt wird, welche Ernährung vom Erkrankten am Besten toleriert wird.

Bei Krebserkrankungen des Verdauungstraktes werden verschiedene protektive Ernährungsfaktoren diskutiert. Neben der Reduktion von Fleisch und Fleischwaren, Fett und Alkohol steht ein reduziertes Krebsrisiko für den Magen-Darmtrakt bei hohem Gemüse- und Früchteverzehr gegenüber. Viskosen Ballaststoffen und resistenter Stärke, die gute Butyratbildner sind, aber auch Nahrungsbestandteilen wie Vitamin C und E oder auch bestimmten Flavonoiden wird eine wichtige Rolle als protektive Faktoren zugesprochen.

Bei der Behandlung von Krebserkrankungen des Magen-Darmtraktes (Operation, Strahlentherapie, Chemotherapie) spielen Nahrungsmittel bisher nur im Sinne einer Therapie-unterstützenden und -ergänzenden Diät eine Rolle. Hier wird v.a. auf die ausreichende Versorgung des Patienten mit Nähr- und Mineralstoffen, Spurenelementen und Vitaminen geachtet.

Als bisher nur wenig genutzte Nahrungsquelle könnte zukünftig Johannisbrot interessant werden. Johannisbrot ist eine Frucht des mediterranen Johannisbrotbaums, die aufgrund ihres hohen natürlichen Kohlenhydratgehaltes ausgesprochen süß schmeckt. Johannisbrot selbst ist eine gute Quelle von Ballaststoffen und Polyphenolen. Wasserlösliche polyphenolreiche Extrakte aus Johannisbrot zeigen eine hohe antioxidative oder auch antimikrobielle Wirksamkeit (Henis et al., Applied Microbiology, Vol. 12, No. 3, 1964, 204-209; Kumazawa et al., J. Agric. Food Chem. 2002, 50, 373-377; Nutrinova, Caromax^{TM} "New Perspectives"; 02/2002). Darüber hinaus ist beschrieben, dass Produkte aus Johannisbrot, hergestellt nach den Verfahren beschrieben in US-A-4,999,197 und US-A-5,330,755, einen antidiarrhötischen Effekt besitzen (auch US-A- 5,043,160). Geröstetes Johannisbrotpulver wurde verschiedentlich als ergänzende diätetische Maßnahme bei Therapie von akuten Diarrhöen, insbesondere bei Kleinkindern, eingesetzt (Loeb et al., J. Pediatr. Gastroenterol. Nutr., 8, 480-485,1989). In der EP-A-0 616 780 wird ein Herstellungsverfahren für ein ballaststoffreiches Präparat aus Johannisbrot beschrieben. Aufgrund des besonderen Herstellungsprozesses erhält man Präparate, die reich an unlöslichen Johannisbrotfasern sind und neben ihrer verdauungsfördernden Wirkung noch weitere gesundheitliche Zusatznutzen wie Cholesterinsenkung zeigen (Pérez-Olleros et al., J. Sci. Food Agric. 79, 173-178, 1999). Für Johannisbrotprodukte, insbesondere unlösliche Johannisbrotfasern, ist bisher nicht beschrieben, ob sie Entzündungsreaktionen oder das Krebsrisiko oder Krebsgeschehen positiv beeinflussen können.

Die bisherigen Lösungsansätze zur Reduzierung des Risikos für entzündliche Erkrankungen oder Krebserkrankungen des Magen-Darmtraktes sind nicht zufriedenstellend. Eines der Grundprobleme gerade im Hinblick auf eine Protektion vor den Erkrankungen ist die aufwändige Ernährungsumstellung aufgrund von Ernährungsempfehlungen. Dies wird vom Verbraucher wenig angenommen, solange er noch nicht wirklich erkrankt ist. Im Sinne einer wirksamen Protektion, die der Verbraucher annimmt, ist es daher notwendig, kompakte, hochwirksame und einfach zu handhabende Lösungen, z.B. in Form von Lebensmitteln des täglich Verzehrs, zu entwickeln. Die bisherigen, als positiv beschriebenen Nahrungsfaktoren wirken v.a. nur schwach unterstützend und oft sehr unspezifisch.

Des weiteren wäre es wünschenswert, durch geeignete Ernährungsmaßnahmen den Einsatz und somit die häufig auftretenden Nebenwirkungen von Medikamenten, insbesondere bei den entzündlichen Darmerkrankungen, aber auch Krebserkrankungen zu reduzieren. Es besteht daher weiterhin der Bedarf nach Lebensmittelkomponenten, die bestimmte Risikofaktoren von entzündlichen Erkrankungen oder Krebserkrankungen des Magen-Darmtraktes senken bzw. verbessern können und die Therapie mit Arzneimitteln sinnvoll unterstützen.

Dies wird gelöst durch ein entzündungshemmendes und chemopräventives Mittel, welches Produkte aus Johannisbrot, insbesondere Johannisbrotfaser, enthält.

Entzündungshemmend im Sinne der Erfindung bedeutet, dass entzündliche Prozesse und Krankheiten durch Interaktion direkt oder indirekt positiv beeinflusst werden und es zu einer Abmilderung von Krankheitssymptomen und Verbesserung des Krankheitsverlaufs kommt. Dabei können Interaktionen mit reaktiven Entzündungsmediatoren wie z. B. unterchlorige Säure (Hypochlorit) eine besondere Rolle spielen. Des weiteren wird darunter aber auch verstanden, dass das Risiko von Entzündungskrankheiten, insbesondere des Gastrointestinaltraktes, herabgesetzt werden kann. Als typische entzündliche Erkrankungen des Gastrointestinaltraktes sind z. B. Darmentzündungen (Enteritis), Gastroenteritis, unspezifisches Reizdarmsyndrom und die chronisch entzündlichen Darmerkrankungen Colitis ulcerosa und Morbus Crohn zu nennen.

Chemopräventiv im Sinne der Erfindung bedeutet, dass durch Interaktion Prozesse innerhalb der Tumorgenese/Kanzerogenese direkt oder indirekt positiv beeinflusst werden und die Manifestation der Krankheit verhindert wird oder das Risiko zu erkranken gesenkt wird. Chemopräventiv bedeutet auch ein Schutz vor genotoxischen und mutagenen Schädigungen, insbesondere DNA-Schädigungen. Dabei können direkte Interaktionen mit DNA-schädigende Agentien und Mutagenen, aber auch Prokanzerogenen, die noch metabolisch aktiviert werden müssen, eine besondere Rolle spielen. Des weiteren wird auch darunter verstanden, dass Krebszellen in ihrem Wachstum gehemmt werden können (antiproliferative Wirkung). Als typische Krebserkrankungen des Gastrointestinaltraktes sind u. a. Magenkrebs und Dickdarmkrebs zu nennen.

Produkte aus Johannisbrot im Sinne der Erfindung sind zum einen das native Johannisbrot (Ceratonia siliqua) selbst oder Folgeprodukte, die aufgrund von technologischen Verfahren wie z. B. mechanische Zerkleinerung, Separierung, Sortierung, thermischer Behandlung, Extraktion, Extrusion, Druckbehandlung usw. gewonnen werden. Entkerntes Johannisbrotfruchtfleisch macht je nach Spezies zwischen 63 und 92 Gew.-% der Gesamtfrucht aus. Die chemische Zusammensetzung von Johannisbrot variiert aufgrund seines biologischen Ursprungs (u. a. abhängig von der Sorte, dem Klima, dem Boden usw.) in relativ großen Schwankungsbreiten. Die Hauptkomponenten des Johannisbrotes sind wasserlösliche Zucker und können normalerweise im Bereich von 30 bis 60 Gew.-%, bevorzugt von 40 bis 50 Gew.-%, enthalten sein.

Die in den erfindungsgemäßen Mitteln eingesetzten Produkte aus Johannisbrot beinhalten alle durch mechanische und physikalische Zerkleinerung, physikochemische Verfahren (Erhitzung, Extraktion usw.) und/oder weitere technologische Behandlungs- und Verfahrensschritte hergestellten Produkte. Insbesondere sind Johannisbrotfasern, bevorzugt hergestellt nach dem Verfahren gemäß EP-A-0 616780, auf die an dieser Stelle ausdrücklich Bezug genommen wird, als Produkte aus Johannisbrot geeignet. Die so gewonnenen Johannisbrotfasern sind weitgehend wasserunlöslich, d. h. sie enthalten noch max. 1 bis 30 Gew.-%, bevorzugt 10 bis 15 Gew.-%, wasserlösliche Bestandteile.

Die erfindungsgemäßen Mittel können neben dem Produkt aus Johannisbrot, insbesondere der Johannisbrotfaser, entsprechend der Anwendungsform auch noch andere Komponenten enthalten. Neben dem direkten Verzehr der Johannisbrotprodukte oder dem direkten Zusatz durch den Verbraucher zu Lebensmitteln und Getränken des allgemeinen Verzehrs können diese auch bei der industriellen Lebensmittelherstellung zugesetzt werden. Im diesem Fall bieten sich für die Johannisbrotprodukte, insbesondere die Johannisbrotfaser, gängige Lebensmittelapplikationen wie z.B. Backwaren, Cerealien, Snack- oder Fruchtriegel oder Getränkepulver an. Darüber hinaus ist auch ein Einsatz in Nahrungsergänzungmittel- oder Arzneimittel-typischer Form wie z.B. Tabletten, Dragees, Kapseln, Sachets oder Granulaten möglich. Die entzündungshemmenden und chemopräventiven Mittel in Nahrungsergänzungsmittel- oder Arzneimittel-typischer Form können übliche Additive wie Lösungsmittel, Füllstoffe, Trägerstoffe wie Methylcellulose, süßende Kohlenhydrate und andere Süßungsmittel, Aromen, Antioxidantien, Farbstoffe usw. enthalten.

Der Einsatz der erfindungsgemäßen Mittel kann auch direkt in Kombination mit den für die Therapie einzusetzenden Arzneimittelwirkstoffen erfolgen. Zur Herstellung dieser Kombinationspräparate verfährt man am besten so, dass man die gewünschten Mengen an Johannisbrotprodukt, insbesondere Johannisbrotfaser, und Wirkstoff miteinander mischt, sprühtrocknet, vom Lösungsmittel befreit, agglomeriert und/oder instantisiert. Des weiteren können alle gängigen lebensmitteltechnologischen, aber auch galenischen Herstellungsverfahren wie z.B. Mischen, Emulgieren, Suspendieren, Dispergieren sowie Pressen, Kneten oder Dragieren verwendet werden.

Die Produkte aus Johannisbrot, insbesondere Johannisbrotfaser, sind in den erfindungsgemäßen Mitteln in Konzentrationen enthalten, die die Risikofaktoren der entsprechenden Krankheiten positiv beeinflussen und somit das Risiko von Entzündungen oder Krebs, insbesondere des Verdauungstraktes, herabsetzen, aber auch die Symptome oder molekulare Prozesse im Verlauf der Krankheiten positiv beeinflussen, abschwächen und somit den Heilungsprozess unterstützen. Die Tagesdosis an Produkten aus Johannisbrot kann je nach Krankheitsbild, jeweiligem Krankheitszustand, aber auch individueller Notwendigkeit dabei im Bereich von 0,1 - 50 g, üblicherweise von 3 - 10 g liegen. Die ggf. vorhandenen Additive bei Nahrungsergänzungsmitteloder Arzneimittel-typischen Applikationen können in Konzentrationen zweckmäßigerweise von 1 - 90 Gew.-%, insbesondere von 10 - 60 Gew.-%, (bezogen auf das Mittel) zugesetzt werden.

Die erfindungsgemäßen Mittel werden zweckmäßigerweise in einer geeigneten, auf die am besten wirkenden Mengenverhältnisse abgestimmten Zubereitung eingesetzt. Dafür kommen für die Produkte aus Johannisbrot, insbesondere der Johannisbrotfaser, v.a. Lebensmittelapplikationen in Frage. Grundsätzlich kommt jedes Lebensmittel, in das die Produkte aus Johannisbrot, insbesondere der Johannisbrotfaser, eingearbeitet werden können, in Frage, wobei sich Grenzen aus den Eigenschaften der Johannisbrotkomponente wie aus dem vorgesehenen Verwendungszweck ergeben. Besonders geeignet sind demnach Lebensmittel auf Getreidebasis wie Backwaren, Cerealien, Snack- und Fruchtriegel, Desserts, Süßspeisen, spezielle Diätzubereitungen wie Getränke und insbesondere Pulvergetränke auf der Basis von Milch, Fruchtkonzentraten oder -pulvern, Kohlenhydraten oder Zuckeralkoholen. Des weiteren können die Produkte aus Johannisbrot, insbesondere der Johannisbrotfaser, als Lebensmittel direkt verzehrt, bei der heimischen Zubereitung von Speisen zugemischt oder in Nahrungsergänzungsmittel appliziert werden. Die gängigen Darreichungsformen von Nahrungsergänzungsmitteln beinhalten Tabletten, Kapseln, aufzulösende pulverförmige Zubereitung oder auch Granulate.

Werden die erfindungsgemäßen Mittel direkt zur Unterstützung einer medikamentösen Therapie verabreicht, kann dies in getrennter Form, aber auch innerhalb eines Produktes geschehen. Für die letztgenannte Variante kommen z. B. pulver- oder tablettenförmige Zubereitungen zur Auflösung, aber auch Kautabletten in Frage. Diese Zubereitungen können außerdem weitere Inhaltsstoffe zur Verbesserung der Auflösung wie lösliche Trägerstoffe, Sprengmittel, und allgemein Farbstoffe, Süßungsmittel wie Saccharose, Glucose, Fructose und andere Kohlenhydrate, Zuckeralkohole wie z. B. Sorbit, Xylit, Maltit und Isomalt oder Süßstoffe wie z. B. Acesulfam-K, Cyclamat, Saccharin, Sucralose oder Aspartam und insbesondere Aromastoffe zur Verbesserung der Akzeptanz enthalten.

Überraschenderweise zeigen die Produkte aus Johannisbrot, insbesondere Johannisbrotfaser, dass sie Entzündungsmediatoren wie z.B. Hypochlorit (unterchlorige Säure), die bei entzündlichen Erkrankungen eine bedeutende Rolle spielen, hochwirksam deaktivieren können und somit deren negativen schädigenden Wirkungen entgegenstehen. Somit können schon im Vorfeld einer potentiellen entzündlichen Erkrankung des Verdauungstraktes verschiedene Entzündungsfaktoren effektiv neutralisiert werden, was mit einer deutlichen Absenkung des Entzündungsrisikos, aber auch einer Milderung der Symptome der Krankheit einhergeht.

Weiterhin wurde überraschenderweise festgestellt, dass die Produkte aus Johannisbrot, insbesondere Johannisbrotfaser, ebenfalls chemopräventive Eigenschaften besitzen. Dies ist im Beispiel der Johannisbrotfaser umso überraschender, da diese überwiegend unlöslich ist und somit viele leicht löslichen und bioverfügbaren Komponenten mittels wässriger Extraktion entfernt werden. Es konnte trotzdem gezeigt werden, dass Johannisbrotfasern Darmzellen effektiv vor DNA-Schädigungen schützen können und somit das Risiko einer möglichen Mutation und des Startes des Kanzerogeneseprozesses herabgesetzt wird. Des weiteren wurde überraschend festgestellt, dass Extrakte von Johannisbrotprodukten, insbesondere Johannisbrotfaser, die unter physiologisch relevanten Bedingungen gewonnen wurden, die Proliferation von Darmkrebszellen hemmen können und somit das Risiko des Auftretens eines Colonkarzinoms herabsetzen.

Die erfindungsgemäßen Mittel erlauben damit eine Absenkung verschiedener Risikofaktoren und Mediatoren von entzündlichen Prozessen, aber auch der Tumorgenese im Magen-Darmtrakt, was schließendlich zum einen das allgemeine Risiko erheblich absenkt, zum anderen die Symptome der Erkrankung erheblich abmildern kann. Im Vergleich zu einer breiten Ernährungsumstellung sind die Effekte der Risikoabsenkung durch die erfindungsgemäßen Mittel erheblich einfacher zu erreichen (z.B. Nahrungsergänzungsmittel oder Riegel mit dem erfindungsgemäßen Mittel), was eine erhebliche Verbesserung zum jetzigen Stand der Technik (u.a. breite Umstellung der Ernährung) darstellt. Des weiteren können die erfindungsgemäßen Mittel die therapeutischen Maßnahmen sinnvoll unterstützen, was zu wünschenswerten Verbesserungen im Krankheitsverlauf führt. Dadurch lässt sich die Dauer und auch die Dosishöhe der Medikation deutlich absenken. Damit lassen sich die mit der Medikation verbundenen Nebenwirkungen drastisch reduzieren, was einen wesentlichen Fortschritt in der medikamentösen Therapie der entzündlichen Erkrankungen des Magen-Darmtraktes darstellt.

Im Folgenden wird die Erfindung anhand von Beispielen erläutert:

### Beispiel 1

### Reaktivität gegenüber dem Entzündungsmediator unterchlorige Säure (HOCI)

Die zytotoxische unterchlorige Säure ist ein spezifisches Produkt Phagozytosekompetenter neutrophiler Granulozyten. Sie dient der Abtötung phagozytierter Bakterien und Viren innerhalb dieser Leukozyten. Die unterchlorige Säure wird aber auch über die Leukozytenmembran in das extrazelluläre Milieu, d.h. in das umgebende Gewebe, sezerniert und zeichnet dort verantwortlich für unerwünschte Reaktionen wie sie an Entzündungsherden beobachtet werden (Schmerz, Rötung, Schwellung).

Zum Nachweis der Reaktivität gegenüber HOCI kann man wie folgt vorgehen. HOCI setzt spezifisch Ethen aus ACC (1-Aminocyclopropan-1-Carboxylsäure) frei, das gaschromatographisch bestimmt wird. Für die Überprüfung der Interaktion der Testsubstanz in diesem Modellsystem wird folgender Ansatz gewählt:

| **Reagenzien** | **Konzentration im Ansatz** |
|---|---|
| Phosphat-Puffer, pH 5,7 | 0.1 M |
| Johannisbrotfasern (Caromax^{TM} ^{*)}, Nutrinova) | 0.005 bis 1.0 mg/2ml |
| HOCI | 25 µM |
| ACC | 1 mM |
| H₂O_{bidest} ad 2ml | |

| | |
|---|---|
| ^{*)} hergestellt nach dem Verfahren gemäß EP-A-0 616 780 | |

Man lässt die unterchlorige Säure mit den Ballaststoffen für 30 Sekunden interagieren und gibt erst dann das Indikatormolekül ACC dazu. Anschließend werden die volumengeeichten, gasdicht verschlossenen Reaktionsgefäße 30 Minuten bei 37 °C inkubiert und die Ethenfreisetzung mittels GC gemessen. Als Positivkontrolle wird 5-Aminosalicylat in Konzentrationen von 0,5 - 100 µM eingesetzt. In der Tabelle. 1 sind die Ergebnisse dieses Tests wiedergegeben.

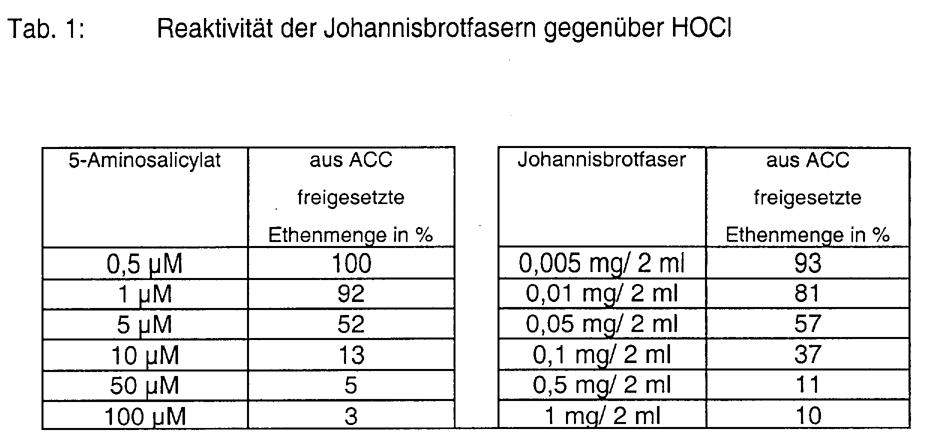

Die Johannisbrotfasern zeigen in diesem Testsystem eine ausgesprochen hohe Reaktivität gegenüber HOCI. Im Vergleich zum entzündungshemmenden Wirkstoff 5-Aminosalicylat zeigt das Johannisbrotprodukt eine sehr ähnliche Reaktivität und kann effektiv diesen Entzündungsmediator inaktivieren und somit die Symptome von Entzündungserkrankungen mildern.

### Beispiel 2

### Protektion von Darmzellen vor genotoxischen Agentien

In diesem Beispiel wird der protektive Effekt von Extrakten der Johannisbrotfaser auf in Kultur gehaltenen Colonzellen gezeigt. Methodisch verfährt man wie folgt:

Die humane Colon-Adenocarcinomzelllinie Caco-2 wurde in Dulbeccos MEM/Nut-Mix F-12 Ham Medium (Gibco) mit 20% FKS (Gibco) und 1% Penicillin/Streptomycin (Gibco) kultiviert. Die Zellen wurden 24 h vor Substanzinkubation in Petrischalen (ø 35 mm) eingesät. Nach Inkubation mit dem physiologischen Extrakt aus Johannisbrotfaser (Stammlösung: 10% w/v Johannisbrotfaser, Caromax™, Nutrinova, in Zellkulturmedium, Arbeitslösungen: 0,001-1%) wurden die Zellen mit PBS-Pufferlösung (phosphate buffered salt solution) gewaschen und nachfolgend mit dem DNAschädigenden Agens Menadion (2 µM) behandelt und abtrypsiniert/zentrifugiert.

Anschließend erfolgte die Prüfung auf die präventive Wirksamkeit der Johannisbrotfaserextrakte.

Die Modulierung der DNA-Schädigung durch die Johannisbrotfaserextrakte im Vergleich zur unbehandelten Kontrolle wurde mittels Einzelzell-Gelelektrophorese (Cometassay) geprüft. Das Ausmaß der DNA-Schädigung wurde über den Anteil an DNA im Kometenschweif quantifiziert (% tail intensity, Tl%). Die unabhängige Grundschädigung wurde stets in Kontrollversuchen miterfasst. Die Ergebnisse sind in Tabelle 2 zusammengefasst:

**Tab. 2:**

| Protektive Wirkung von Johannisbrotextrakt (JBE) auf Caco-2-Zellen | |
|---|---|
| **Substanz** | **% TI** |
| Kontrolle | 2,6 |
| Menadion 2 µM | 13,6 |
| JBE 0,001 % | 13,9 |
| JBE 0,01 % | 12,0 |
| JBE 0,03 % | 7,1 |
| JBE 0,1 % | 3,2 |
| JBE 0,3 % | 5,1 |
| JBE 1 % | 7,6 |

Die durch Menadion verursachten DNA-Schädigungen, ausgedrückt durch einen Tl-Wert von 13,6, lassen sich durch Johannisbrotfaserextrakte je nach Konzentration bis auf das Kontrollniveau (keine induzierten DNA-Schäden) absenken. Damit weist das getestete Johannisbrotprodukt eine außerordentlich ausgeprägte Wirksamkeit gegenüber DNA-Schädigungen in Darmzellen auf. Durch diesen protektiven Effekt werden Krebsprozess-initiierende DNA-Schädigungen reduziert und damit das Krebsrisiko reduziert.

### Beispiel 3

### Antiproliferativer Effekt von Johannisbrotprodukten auf Darmkrebszellen

In diesem Beispiel wird der antiproliferative Effekt von Extrakten der Johannisbrotfaser auf in Kultur gehaltenen HT29-Zellen gezeigt. Methodisch verfährt man wie folgt:

Die humane Kolonkrebszelllinie HT29 wurde in Dulbeccos Modified Eagle Medium (Gibco) mit 10% FKS (Gibco) und 5% Penicillin/Streptomycin (Gibco) kultiviert. Zur Bestimmung der Proliferation werden die Zellen in 96well Platten ausgesät. Die Inkubation mit dem Johannisbrotfaserextrakt (Stammlösung: 10% w/v Johannisbrotfaser, Caromax^{TM}, Nutrinova, in Zellkulturmedium, Arbeitslösungen: 0,05-2%) erfolgte nach 24 h. Nach einer Inkubationszeit von 72 h wird der DNA-Gehalt als Maß für die Proliferation mittels DAPI (4',6-Diamidino-2-phenylindol-dihydrochlorid)-Färbung bestimmt.

Die Ergebnisse der Proliferationstests sind in Tabelle 3 zusammengefasst:

**Tab. 3:**

| Antiproliferative Wirkung von Johannisbrotextrakt (JBE) bei HT29-Zellen | |
|---|---|
| **JBE Konzentration in %** | **Proliferation in %** |
| Kontrolle | 100 |
| 0,05 | 97 |
| 0,1 | 93 |
| 0,2 | 85 |
| 0,3 | 69 |
| 0,4 | 55 |
| 0,6 | 32 |
| 1 | 15 |
| 2 | 9 |

Johannisbrotfaserextrakte modulieren signifikant das Wachstum von humanen Kolonkrebszelllinien. Der IC50-Wert (Wert bei dem 50% der Zellen nicht mehr wachsen) liegt bei 0,42 Gew.-%. Somit haben Johannisbrotprodukte einen signifikanten Einfluss auf die Prozesse bei der Kolonkarzinogenese.

### Beispiel 4

### Kautablette

Die Kautabletten wurden nach folgender Rezeptur erstellt:

| | |
|---|---|
| Johannisbrotfaser (Caromax^{TM}, Nutrinova) | 2 g |
| Sorbit (Roquette) | 1,4 g |
| Magnesiumstearat (Merck) | 15 mg |
| Acesulfam K (Sunett®, Nutrinova) | 12 mg |
| Aspartam (Holland Sweetener Corp.) | 12 mg |
| Schokoladenaroma (Danisco) | q.s. |

Die Zutaten der Kautabletten werden in üblicher Weise trocken vermischt und gepresst.

### Beispiel 5:

### Fruchtriegel

Die Fruchtriegel wurden nach folgender Rezeptur hergestellt:

| | |
|---|---|
| Fruchtzubereitung Orange (Zentis) | 6 g |
| Marzipan | 5 g |
| Orangeat | 5 g |
| Johannisbrotfaser (Caromax™, Nutrinova) | 5 g |
| Feigen | 3 g |
| Sultaninen | 2 g |
| Äpfel | 2 g |

Alle Zutaten werden miteinander vermischt und fein zerkleinert. Danach wird die Masse in Form gepresst, und die entstandenen Riegelkerne werden mit Oblatenblättern von oben und unten bedeckt.

## Patentansprüche

1. Johannisbrotprodukt zur Verwendung als Nahrungsergänzungsmittel.

2. Johannisbrotprodukt zur Verwendung als entzündungshemmendes Nahrungsergänzungsmittel.

3. Johannisbrotprodukt zur Verwendung als chemopräventives Nahrungsergänzungsmittel.

4. Johannisbrotprodukt nach einem der Ansprüche 1 bis 3, **dadurch gekennzeichnet, dass** es entkerntes Johannisbrotfruchtfleisch enthält.

5. Johannisbrotprodukt nach einem der Ansprüche 1 bis 3, **dadurch gekennzeichnet, dass** es Johannisbrotfaser enthält.

6. Johannisbrotprodukt nach einem der Ansprüche 1 bis 3, **dadurch gekennzeichnet, dass** es aus Johannisbrotfaser besteht.

7. Johannisbrotprodukt, nach Anspruch 5 oder 6, **dadurch gekennzeichnet, dass** die Johannisbrotfasern noch 1 bis 30 Gew.-% wasserlösliche Bestandteile enthalten.

8. Johannisbrotprodukt nach einem der Ansprüche 1 bis 7, **dadurch gekennzeichnet, dass** es einen zusätzlichen Wirkstoff enthält.

9. Johannisbrotprodukt nach einem der Ansprüche 1 bis 8 in Form eines Lebensmittels.

10. Johannisbrotprodukt nach einem der Ansprüche 1 bis 8 in Form eines Getränks.

11. Johannisbrotprodukt nach einem der Ansprüche 1 bis 8 in Form eines Lebensmittelzusatzstoffes.

12. Johannisbrotprodukt nach einem der Ansprüche 1 bis 8 in Form eines Tierfuttermittels.

13. Johannisbrotprodukt nach Anspruch 8 in Form eines Arzneimittels.

14. Johannisbrotprodukt nach Anspruch 8 in Form eines Tierarzneimittels.

15. Verwendung eines Johannisbrotproduktes zur Herstellung eines entzündungshemmend wirkenden Nahrungsergänzungsmittels.

16. Verwendung eines Johannisbrotproduktes zur Herstellung eines chemopräventiv wirkenden Nahrungsergänzungsmittels.

17. Verfahren zur Herstellung eines entzündungshemmend wirkenden Nahrungsergänzungsmittels, **gekennzeichnet durch** die Verwendung eines Johannisbrotproduktes.

18. Verfahren zur Herstellung eines chemopräventiv wirkenden Nahrungsergänzungsmittels, **gekennzeichnet durch** die Verwendung eines Johannisbrotproduktes.
